# EUROPÄISCHE PATENTANMELDUNG

(11) **EP 3 536 385 A2**
(43) Veröffentlichungstag der Anmeldung: **11.09.2019**
(21) Anmeldenummer: 19166093.5
(22) Anmeldetag: 09.12.2014
(51) Int. Cl.: A61Q 5/02, A61Q 5/12, A61K 8/42, A61K 8/31, A61K 8/46

(54) **HAARBEHANDLUNGSMITTEL ENTHALTEND CARBONSÄURE-N-ALKYLPOLYHYDROXYALKYLAMIDE**

(30) Priorität: 10.12.2013 DE 102013225373
(62) Teilanmeldung aus: 14814769.7
(71) Anmelder: Clariant International Ltd, 4132 Muttenz (CH)
(72) Erfinder: Scheunemann, Volker, 21339 Lüneburg (DE); Wiesche, Erik Schulze zur, 22453 Hamburg (DE)
(74) Vertreter: Kampen, Daniela

(57) **Zusammenfassung**

Die vorliegende Erfindung betrifft ein Haarbehandlungsmittel, enthaltend:
a) mindestens ein Carbonsäure-N-alkylpolyhydroxyalkylamid der Formel (I) 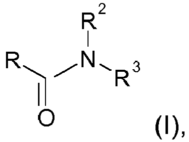 worin
R
für einen aliphatischen Alkylrest mit 16 bis 18 Kohlenstoffatomen steht,
R²
für einen Alkylrest oder einen Hydroxyalkylrest mit 1 bis 4 Kohlenstoffatomen steht,
R³
für einen linearen oder verzweigten Polyhydroxyalkylrest mit 3 bis 12 Kohlenstoffatomen und 3 bis 10 Hydroxylgruppen steht, und
b) mindestens ein weiteres Tensid, ausgewählt aus der Gruppe der
(i) Alkylethersulfate mit 8 bis 18 Kohlenstoffatomen und 1 bis 10 Oxyethylgruppen;
(ii) Alkylsulfate mit 8 bis 18 Kohlenstoffatomen;
(iii) Alkylpolyglucoside;
(iv) amphoteren Tenside;
sowie deren Mischungen,
welches eine hervorragende Reinigungswirkung aufweist und zu einer verbesserten Trocken- und Nasskämmbarkeit sowie einem verbesserten Griff führt.

## Beschreibung

Die vorliegende Erfindung betrifft das technische Gebiet der Haarreinigung und der Haarpflege.

Insbesondere betrifft die vorliegende Erfindung ein Haarbehandlungsmittel, enthaltend mindestens ein spezielles Carbonsäure-N-alkylpolyhydroxyalkylamid und mindestens ein weiteres Tensid, ausgewählt aus der Gruppe der
(i) Alkylethersulfate mit 8 bis 18 Kohlenstoffatomen und 1 bis 10 Oxyethylgruppen;
(ii) Alkylsulfate mit 8 bis 18 Kohlenstoffatomen;
(iii) Alkylpolyglucoside;
(iv) amphoteren Tenside;
sowie deren Mischungen.

Weiterhin betrifft die vorliegende Erfindung die Verwendung des erfindungsgemäßen Haarbehandlungsmittels zur Reinigung und Pflege von Kopfhaut und Haaren.

Schließlich betrifft die vorliegende Erfindung ein Verfahren zur Reinigung und Pflege von Kopfhaut und Haaren, wobei ein erfindungsgemäßes Haarbehandlungsmittel auf das trockene oder gegebenenfalls zuvor gewaschene und/oder nasse Haar aufgetragen und nach einer gewissen Zeitdauer gegebenenfalls ausgespült wird, wobei nach der Anwendung des Haarbehandlungsmittels eine verbesserte Trocken- und Nasskämmbarkeit sowie ein verbesserter Griff auftreten.

Haarbehandlungsmittel in Form von Haarreinigungsmitteln müssen eine gute Reinigungswirkung aufweisen, um zuverlässig Verunreinigungen von den Haaren und der Kopfhaut zu entfernen. Weiterhin sollten die Haarbehandlungsmittel, insbesondere die darin enthaltenen Tensidsysteme, keine Ablagerungen auf den Haaren verursachen, da hierdurch Haareigenschaften, wie die Kämmbarkeit und der Griff, negativ beeinflusst werden.

Da die Haare bei der Haarreinigung beansprucht werden, werden den meisten Haarbehandlungsmitteln in Form von Haarreinigungsmitteln zusätzlich Pflegeprodukte und Pflegestoffe mit möglichst lang anhaltender Wirkung zugesetzt, um eine zu starke Beanspruchung der Haare zu vermeiden. Derartige Pflegeprodukte und Pflegestoffe beeinflussen die natürliche Struktur und die Eigenschaften der Haare. So können anschließend an solche Behandlungen beispielsweise die Nass- und Trockenkämmbarkeit des Haares, der Halt und die Fülle des Haares optimiert sein oder die Haare vor erhöhtem Spliss geschützt sein.

Es ist daher seit langem üblich, Haarreinigungsmitteln spezielle Wirkstoffe, beispielsweise quaternäre Ammoniumsalze oder spezielle Polymere, zuzusetzen. Durch diese Behandlung werden je nach Formulierung die Kämmbarkeit, der Halt und die Fülle der Haare verbessert und die Splissrate verringert.

Es besteht nach wie vor das Bedürfnis, Haarbehandlungsmittel bereitzustellen, welche eine hohe Reinigungs- und/oder Pflegeleistung aufweisen und sich einfach anwenden lassen. Insbesondere besteht ein Bedarf an Haarbehandlungsmitteln, deren Tensidsysteme eine vorteilhafte Wirkung auf Haareigenschaften, wie Kämmbarkeit und Griff, haben und nicht zu einer ungünstigen Wechselwirkung mit weiteren Pflege- und Inhaltsstoffen führen.

Der vorliegenden Erfindung lag die Aufgabe zugrunde, Haarbehandlungsmittel bereitzustellen, welche einfach anzuwenden sind und welche eine gute Reinigungsleistung und/oder Pflegeleistung aufweisen. Insbesondere sollen die Trocken- und Nasskämmbarkeit und der Haargriff nach der Anwendung verbessert sein. Eine weitere Aufgabe der vorliegenden Erfindung lag in der Bereitstellung von Haarpflegemitteln, welche eine hohe Stabilität selbst während langer Lagerungsdauern aufweisen.

Es wurde nun überraschend gefunden, dass Haarbehandlungsmittel, welches ein spezielles Tensidsystem aus Carbonsäure-N-alkylpolyhydroxyalkylamiden und weiteren Tensiden enthalten, eine hohe Reinigungsleistung aufweisen und darüber hinaus dieses Tensidsystem weder einen negativen Einfluss auf die Haareigenschaften noch eine ungünstige Wechselwirkung mit anderen Pflege- und Inhaltsstoffen aufweist. Weiterhin führen die erfindungsgemäßen Haarbehandlungsmittel zu guten Kämmeigenschaften von nassen und trockenen Haaren und zu verbessertem Haargriff. Darüber hinaus ist eine problemlose Einarbeitung des speziellen Tensidsystems und somit eine kostengünstige und einfache Herstellung möglich. Schließlich führt das spezielle Tensidsystem auch zu einer hohen Lagerstabilität der erfindungsgemäßen Haarbehandlungsmittel.

Gegenstand der vorliegenden Erfindung ist somit ein Haarbehandlungsmittel, enthaltend:
a) mindestens ein Carbonsäure-N-alkylpolyhydroxyalkylamid der Formel (I) 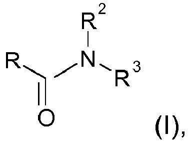 worin
   - R: für einen aliphatischen Alkylrest mit 16 bis 18 Kohlenstoffatomen steht,
   - R²: für einen Alkylrest oder einen Hydroxyalkylrest mit 1 bis 4 Kohlenstoffatomen steht,
   - R³: für einen linearen oder verzweigten Polyhydroxyalkylrest mit 3 bis 12 Kohlenstoffatomen und 3 bis 10 Hydroxylgruppen steht, und
b) mindestens ein weiteres Tensid, ausgewählt aus der Gruppe der
   (i) Alkylethersulfate mit 8 bis 18 Kohlenstoffatomen und 1 bis 10 Oxyethylgruppen;
   (ii) Alkylsulfate mit 8 bis 18 Kohlenstoffatomen;
   (iii) Alkylpolyglucoside;
   (iv) amphoteren Tenside;
sowie deren Mischungen.

Die erfindungsgemäßen Haarbehandlungsmittel weisen aufgrund der Verwendung von speziellen Carbonsäure-N-alkylpolyhydroxyalkylamiden als Cotensiden ein hohes Schaumvermögen auf und führen zu einer hervorragenden Reinigungsleistung. Weiterhin führt der Einsatz des Cotensids zu einer verbesserten Konditionierung der Haare, insbesondere zu einer verbesserten Kämmbarkeit und einem verbesserten Griff. Darüber hinaus resultiert die Verwendung von Carbonsäure-N-alkylpolyhydroxyalkylamide als Cotenside nicht in einer Verminderung der Wirksamkeit haarpflegender Inhaltsstoffe, so dass bei Einsatz von pflegenden Wirkstoffen die Konditionierung der Haare weitergehend verbessert werden kann. Zudem können die Carbonsäure-N-alkylpolyhydroxyalkylamide in allgemein übliche Formulierungen eingearbeitet werden, ohne die Stabilität, insbesondere die Lagerstabilität, der Haarbehandlungsmittel signifikant zu beeinflussen.

Haarbehandlungsmittel im Sinne der vorliegenden Erfindung sind beispielsweise Haarshampoos, Haarkonditionierer, konditionierende Shampoos, Haarspülungen, Haarkuren, Haarpackungen, Haar-Tonics, Haarfärbeshampoos oder deren Kombinationen. Insbesondere werden das Haar konditionierende Zusammensetzungen, wie konditionierende Shampoos, Haarspülungen, Haarkuren, Haarpackungen, Haaröle und- lotionen sowohl als leave-on, also als auf dem Haar bis zur nächsten Haarwäsche verbleibende Produkte, als auch als rinse-off, also wenige Sekunden bis wenige Stunden nach der Anwendung wieder auszuspülende Produkte, unter den erfindungsgemäßen Haarbehandlungsmitteln verstanden.

Unter Kämmbarkeit wird erfindungsgemäß sowohl die Kämmbarkeit der nassen Faser, als auch die Kämmbarkeit der trockenen Faser verstanden.

Weiterhin wird als Griff im Rahmen der vorliegenden Erfindung die Taktilität eines Faserkollektivs verstanden, wobei der Fachmann sensorisch die Parameter Fülle und Geschmeidigkeit des Kollektivs fühlt und bewertet.

Die Angabe der Gesamtmenge in Bezug auf die Komponenten des Haarbehandlungsmittels bezieht sich vorliegend - sofern nicht anderes angegeben ist - auf die Gesamtmenge an Aktivsubstanz der jeweiligen Komponente.

Besonders gute Ergebnisse werden im Rahmen der vorliegenden Erfindung erhalten, wenn das Haarbehandlungsmittel mindestens ein Carbonsäure-N-alkylpolyhydroxyalkylamid der Formel (la) und/oder der Formel (Ib) 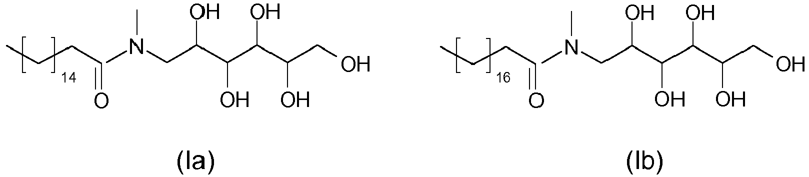 enthält. Durch den Einsatz dieser speziellen Carbonsäure-N-alkylpolyhydroxyalkylamide als Cotenside werden ein verbessertes Schaumvermögen und eine verbesserte Reinigungsleistung erhalten. Weiterhin führen diese speziellen Carbonsäure-N-alkylpolyhydroxyalkylamide zu einer Verbesserung der Haareigenschaften, wie der Kämmbarkeit und des Griffs. Darüber hinaus lassen sich diese Cotenside hervorragend in Formulierungen Einarbeiten und ermöglichen so eine einfach und kostengünstige Herstellung der erfindungsgemäßen Haarbehandlungsmittel. Schließlich führt die Verwendung dieser speziellen Cotenside nicht zu einer verminderten Lagerstabilität der Haarbehandlungsmittel.

Gemäß einer bevorzugten Ausführungsform der vorliegenden Erfindung enthält das Haarbehandlungsmittel das mindestens eine Carbonsäure-N-alkylpolyhydroxyalkylamid der Formel (I) in einer Gesamtmenge von 0,01 bis 30 Gew.-%, vorzugsweise von 0,1 bis 20 Gew.-%, bevorzugt von 0,3 bis 15 Gew.-%, weiter bevorzugt von 0,5 bis 10 Gew.-%, noch weiter bevorzugt von 0,7 bis 8 Gew.-%, insbesondere von 1,0 bis 5,0 Gew.-%, bezogen auf das Gesamtgewicht des Haarbehandlungsmittels. Die Verwendung des speziellen Carbonsäure-N-alkylpolyhydroxyalkylamids in den vorstehend genannten Mengen resultiert zum einen in einer guten Reinigungsleistung und zum anderen in einem positiven Einfluss auf die Haareigenschaften, wie die Kämmbarkeit und den Griff. Darüber hinaus wird durch den Einsatz der zuvor genannten Mengen des speziellen Carbonsäure-N-alkylpolyhydroxyalkylamids ein negativer Einfluss auf haarpflegende Wirkstoffe vermieden und somit eine weitergehend verbesserte konditionierende Wirkung erreicht.

Erfindungsgemäß bevorzugt wird das mindestens eine weitere Tensid ausgewählt aus der Gruppe der
(i) Natriumlaurethsulfate;
(ii) Natriumlaurylsulfate;
(iii) Alkylpolyglucoside der Formel

   R⁴O-[G]ₚ

   in welcher
   - R⁴: für einen Alkyl- und/oder Alkenylrest mit 4 bis 22 Kohlenstoffatomen,
   - G: für einen Zuckerrest mit 5 oder 6 Kohlenstoffatomen und
   - p: für Zahlen von 1 bis 10 steht;
(iv) C₈₋₁₈-Alkylamido(C₁₋₄)-alkylbetaine;
sowie deren Mischungen.

Eine besonders hohe Reinigungsleistung wird erreicht, wenn das spezielle Carbonsäure-N-alkylpolyhydroxyalkylamid in Kombination mit den vorstehend genannten Tensiden eingesetzt wird. Gleichzeitig führt der Einsatz der Kombination der vorstehend genannten Tenside mit dem speziellen Carbonsäure-N-alkylpolyhydroxyalkylamid zu einer verbesserten Nass- und Trockenkämmbarkeit sowie zu einem verbesserten Griff. Weiterhin weist die Kombination keine ungünstigen Wechselwirkungen mit weiteren Pflegestoffen auf, so dass durch deren Verwendung die konditionierenden Eigenschaften des erfindungsgemäßen Haarbehandlungsmittels weitergehend gesteigert werden können.

Besonders gute Ergebnisse im Hinblick auf die Reinigungsleistung und das Schaumvermögen werden erhalten, wenn das mindestens eine weitere Tensid in einer Gesamtmenge von 0,01 bis 50 Gew.-%, vorzugsweise von 0,1 bis 40 Gew.-%, bevorzugt von 2 bis 30 Gew.-%, weiter bevorzugt von 5 bis 20 Gew.-%, insbesondere von 8 bis 15 Gew.-%, bezogen auf das Gesamtgewicht des Haarbehandlungsmittels, enthalten ist. Der Einsatz des mindestens einen weiteren Tensids in den zuvor genannten Mengen führt in Kombination mit dem speziellen Carbonsäure-N-alkylpolyhydroxyalkylamid zu einer hervorragenden Reinigung der Haare und der Kopfhaut und vermeidet einen negativen Einfluss auf die Haareigenschaften, die weiteren Pflege- und Inhaltsstoffe und die Lagerstabilität des Haarbehandlungsmittels.

Gemäß einer weiteren Ausführungsform der vorliegenden Erfindung enthält das Haarbehandlungsmittel ein Gewichtsverhältnis der Gesamtmenge des mindestens einen Carbonsäure-N-alkylpolyhydroxyalkylamids (a) zu der Gesamtmenge des mindestens einen weiteren Tensids (b) von 1 : 15 bis 1 : 1, vorzugsweise von 1 : 12 bis 1 : 2, bevorzugt von 1 : 8 bis 1 : 2,5, insbesondere von 1 : 6 bis 1 : 3. Der Einsatz des mindestens einen speziellen Carbonsäure-N-alkylpolyhydroxyalkylamids und des mindestens einen Tensids in diesen Gewichtsverhältnissen resultiert zum einen in einem hohen Schaumvermögen und in einer hervorragenden Reinigungsleistung der erfindungsgemäßen Haarbehandlungsmittel. Zum anderen wird durch den Einsatz dieser Gewichtsverhältnisse die Trocken- und Nasskämmbarkeit sowie der Griff der Haare verbessert. Darüber hinaus treten bei Verwendung der Gewichtsverhältnisse keine ungünstigen Wechselwirkungen mit weiteren pflegenden Wirkstoffe in dem erfindungsgemäßen Haarbehandlungsmittel auf, so dass die Kämmbarkeit und der Griff bei Einsatz zusätzlicher konditionierender Wirkstoffe weitergehend verbessert werden können. Darüber hinaus führt dieses Gewichtsverhältnis zu lagerstabilen Formulierungen.

Eine besonders bevorzugte Ausführungsform der vorliegenden Erfindung ist demnach ein Haarbehandlungsmittel, enthaltend:
a) mindestens ein Carbonsäure-N-alkylpolyhydroxyalkylamid der Formel (la) und/oder der Formel (Ib) 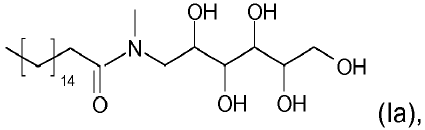 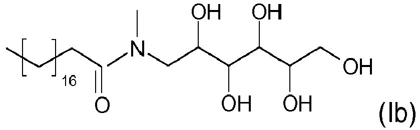 in einer Gesamtmenge von 2,5 bis 3,5 Gew.-%, bezogen auf das Gesamtgewicht des Haarbehandlungsmittels, und
b) mindestens ein weiteres Tensid, ausgewählt aus Natriumlaurethsulfat und/oder C₁₂-Alkylamido(C₃)-alkylbetain in einer Gesamtmenge von 2 bis 12 Gew.-%, bezogen auf das Gesamtgewicht des Haarbehandlungsmittels.

Weiterhin werden besonders gute Ergebnisse erhalten, wenn ein Haarbehandlungsmittel, enthaltend:
a) mindestens ein Carbonsäure-N-alkylpolyhydroxyalkylamid der Formel (la) und/oder der Formel (Ib) 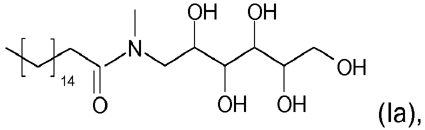 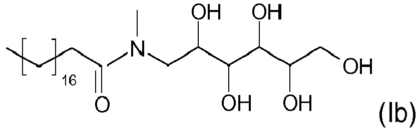 in einer Gesamtmenge von 2,5 bis 3,5 Gew.-%, bezogen auf das Gesamtgewicht des Haarbehandlungsmittels, und
b) mindestens ein weiteres Tensid, ausgewählt aus Natriumlaurylsulfat und/oder C₁₂-Alkylamido(C₃)-alkylbetain in einer Gesamtmenge von 2 bis 12 Gew.-%, bezogen auf das Gesamtgewicht des Haarbehandlungsmittels,
eingesetzt wird.

Darüber hinaus werden ebenfalls besonders gute Ergebnisse erhalten, wenn ein Haarbehandlungsmittel nach einem der vorangehenden Ansprüche, enthaltend:
a) mindestens ein Carbonsäure-N-alkylpolyhydroxyalkylamid der Formel (la) und/oder der Formel (Ib) 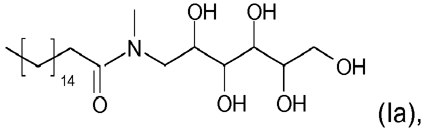 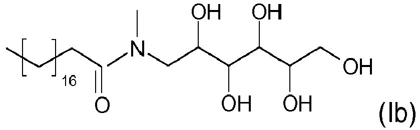 in einer Gesamtmenge von 2,5 bis 3, 5 Gew.-%, bezogen auf das Gesamtgewicht des Haarbehandlungsmittels, und
b) mindestens ein weiteres Tensid, ausgewählt aus Natriumlaurethsulfat und/oder Natriumlaurylsulfat und/oder C₁₂-Alkylamido(C₃)-alkylbetain in einer Gesamtmenge von 2 bis 15 Gew.-%, bezogen auf das Gesamtgewicht des Haarbehandlungsmittels,
eingesetzt wird.

Die vorstehend genannten drei besonders bevorzugten Ausführungsformen, welche eine Kombination von äußerst speziellen Carbonsäure-N-alkylpolyhydroxyalkylamiden mit weiteren Tensiden in bestimmten Mengen enthalten, führen zu einer hervorragenden Reinigungsleistung. Weiterhin weisen die zuvor genannten Kombinationen von Carbonsäure-N-alkylpolyhydroxyalkylamiden und speziellen Tensiden keine negativen Wirkung auf die Haareigenschaften auf und führen darüber hinaus nicht zu ungünstigen Wechselwirkungen mit weiteren Inhalts- und Pflegestoffen. Zudem führen diese Kombinationen nicht zu einer Verringerung der Lagerstabilität der erfindungsgemäßen Haarbehandlungsmittel.

Neben den vorgenannten Bestandteilen können die Haarbehandlungsmittel eine Reihe weiterer Wirk- und Inhaltsstoffe enthalten. Bevorzugt werden den Haarbehandlungsmitteln weitere Wirkstoffe zugesetzt, welche zusätzliche kosmetische Pflegeeigenschaften aufweisen, um die Konditionierung der Haare während des Reinigungsvorgangs zu unterstützen.

Um die pflegenden Eigenschaften der erfindungsgemäßen Haarbehandlungsmittel weitergehend zu verbessern, kann es daher vorgesehen sein, dass das Haarbehandlungsmittel weiterhin mindestens einen haar- und/oder hautkonditionierenden Wirkstoff enthält, wobei der Wirkstoff ausgewählt ist aus der Gruppe der Ölkomponenten, kationischen Polymere, Pflanzenextrakte und/oder Feuchthaltemittel.

Erfindungsgemäß geeignete Ölkomponenten können ausgewählt sein aus mineralischen, natürlichen oder synthetischen Ölkomponenten wie Petrolatum, Paraffinen, Silikonen, Alkoholen, Fettsäureestern, natürlichen Ölen pflanzlichen und tierischen Ursprungs sowie deren Mischungen, und werden in einer Gesamtmenge von 0,005 bis 20 Gew.-%, bevorzugt von 0,01 bis 10 Gew.-%, besonders bevorzugt von 0,05 bis 5 Gew.-%, insbesondere von 0,2 bis 3 Gew.-%, bezogen auf das Gesamtgewicht des Haarbehandlungsmittels, eingesetzt.

Unter dem Begriff der Silikone versteht der Fachmann verschiedene Strukturen siliciumorganischer Verbindungen, welche in den erfindungsgemäßen Haarbehandlungsmittel in einer Gesamtmenge von 0,01 bis 3 Gew.-%, bevorzugt von 0,05 bis 2 Gew.-%, insbesondere von 0,1 bis 1 Gew.-%, bezogen auf das Gesamtgewicht des Haarbehandlungsmittels, enthalten sein können.

Bevorzugt können die Silikone ausgewählt sein aus mindestens einem Vertreter der Gruppe siliciumorganischer Verbindungen, welche gebildet wird aus:
(i) Polyalkylsiloxanen, Polyarylsiloxanen, Polyalkylarylsiloxanen, welche flüchtig oder nicht flüchtig, geradkettig, verzweigt oder zyklisch, vernetzt oder nicht vernetzt sind;
(ii) Polysiloxanen, welche in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)-Polyoxyalkylen(B)-Blockcopoylmeren vom Typ (A-B)ₙ mit n > 3;
(iv) gepfropften Silikonpolymeren mit nicht silikonhaltigem, organischen Grundgerüst, die aus einer organischen Hauptkette bestehen, welche aus organischen Monomeren gebildet wird, die kein Silikon enthalten, auf die in der Kette sowie gegebenenfalls an mindestens einem Kettenende mindestens ein Polysiloxanmakromer gepfropft wurde;
(v) gepfropften Silikonpolymeren mit Polysiloxan-Grundgerüst, auf das nicht silikonhaltige, organische Monomere gepfropft wurden, welche eine Polysiloxan-Hauptkette aufweisen, auf die in der Kette sowie gegebenenfalls an mindestens einem ihrer Enden mindestens ein organisches Makromer gepfropft wurde, das kein Silikon enthält, wie beispielsweise das unter der INCI-Bezeichnung Bis-PEG/PPG-20/20 Dimethicone vertriebene Handelsprodukt Abil B 8832 der Firma Degussa;
(vi) oder deren Mischungen.

In einer Ausführungsform der vorliegenden Erfindung ist das Konditioniermittel ein konditionierendes Silikon mit einer Viskosität von 20.000 bis 120.000 mPa·s, vorzugsweise von 40.000 bis 80.000 mPa·s.

Besonders bevorzugt ist dabei das konditionierende Silikon ausgewählt aus Dimethiconen, Amodimethiconen oder Dimethiconolen.

Als Fettalkohole können gesättigte, ein- oder mehrfach ungesättigte, verzweigte oder unverzweigte Fettalkohole mit C₆-C₃₀-, bevorzugt C₁₀-C₂₂- und ganz besonders bevorzugt C₁₂-C₂₂-Kohlenstoffatomen eingesetzt werden. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren mit C₆-C₃₀-Kohlenstoffatomen ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnussöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol®, z. B. Stenol® 1618 oder Lanette®, z. B. Lanette® O oder Lorol®, z. B. Lorol® C8, Lorol® C14, Lorol® C18, Lorol® C8-18, HD-Ocenol®, Crodacol®, z. B. Crodacol® CS, Novol®, Eutanol® G, Guerbitol® 16, Guerbitol® 18, Guerbitol® 20, Isofol® 12, Isofol® 16, lsofol® 24, Isofol® 36, Isocarb® 12, Isocarb® 16 oder Isocarb® 24 käuflich zu erwerben. Selbstverständlich können erfindungsgemäß auch Wollwachsalkohole, wie sie beispielsweise unter den Bezeichnungen Corona®, White Swan®, Coronet® oder Fluilan® käuflich zu erwerben sind, eingesetzt werden.

Erfindungsgemäß geeignete Fettalkohole werden in den Haarbehandlungsmitteln in einer Gesamtmenge von 0,01 bis 3 Gew.-%, bevorzugt von 0,05 bis 2 Gew.-%, insbesondere von 0,1 bis 1 Gew.-%, bezogen auf das Gesamtgewicht des Haarbehandlungsmittels, eingesetzt.

Als natürliche oder synthetische Wachse können erfindungsgemäß feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs und Microwachse aus PE- oder PP eingesetzt werden. Derartige Wachse sind beispielsweise erhältlich über die Fa. Kahl & Co., Trittau.

Zu den natürlichen und synthetischen kosmetischen Ölkörpern sind beispielsweise zu zählen:
- flüssige Paraffinöle, Isoparaffinöle und synthetische Kohlenwasserstoffe sowie Di-n-alkylether mit insgesamt zwischen 12 bis 36 Kohlenstoffatomen, insbesondere 12 bis 24 Kohlenstoffatomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.
- Esteröle. Unter Esterölen werden im Rahmen der vorliegenden Erfindung die Ester von C₆-C₃₀-Fettsäuren mit C₂-C₃₀- Fettalkoholen verstanden. Bevorzugt sind die Monoester der Fettsäuren mit Alkoholen mit 2 bis 24 Kohlenstoffatomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, welche z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, welche z. B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit® IPM), Isononansäure-C₁₆₋₁₈-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkohol-caprinat/- caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).
- Dicarbonsäureester, wie Di-n-butyladipat, Di-(2-ethylhexyl)-adipat, Di-(2-ethylhexyl)-succinat und Di-isotridecylacelaat sowie Diolester, wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, Glycerincarbonat oder Dicaprylylcarbonat (Cetiol® CC),
- Trifettsäureester von gesättigten und/oder ungesättigten linearen und/oder verzweigten Fettsäuren mit Glycerin,
- Fettsäurepartialglyceride, insbesondere Monoglyceride, Diglyceride und deren technische Gemische. Bei der Verwendung technischer Produkte können herstellungsbedingt noch geringe Mengen Triglyceride enthalten sein. Die Partialglyceride weisen vorzugsweise die Formel (II) auf 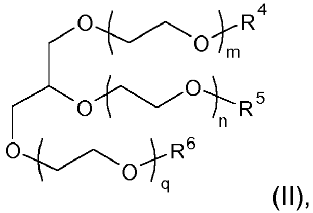 in welcher
   R⁴, R⁵ und R⁶, jeweils unabhängig voneinander, für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R⁴ für einen Acylrest und R⁵ und R⁶ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

In einer besonders bevorzugten Ausführungsform der Erfindung wird als Ölkomponente ein pflanzliches Öl eingesetzt.

Als natürliche Öle kommen beispielsweise Amaranthsamenöl, Aprikosenkernöl, Arganöl, Avocadoöl, Babassuöl, Baumwollsaatöl, Borretschsamenöl, Camelinaöl, Distelöl, Erdnussöl, Granatapfelkernöl, Grapefruitsamenöl, Hanföl, Hagebuttenkernöl, Haselnussöl, Holundersamenöl, Johannisbeersamenöl, Jojobaöl, Kakaobutter, Leinöl, Macadamianussöl, Maiskeimöl, Mandelöl, Marulaöl, Nachtkerzenöl, Olivenöl, Palmöl, Pfirsichkernöl, Rapsöl, Reisöl, Sanddornfruchtfleischöl, Sanddornkernöl, Sesamöl, Sheabutter, Sojaöl, Sonnenblumenöl, Traubenkernöl, Walnussöl oder Wildrosenöl in Frage. Besonders bevorzugt sind erfindungsgemäß Avocadoöl, Aprikosenkernöl, Hagebuttenkernöl, Jojobaöl, Kakaobutter, Mandelöl, Olivenöl, Pfirsichkernöl, Sheabutter, Sonnenblumenöl und Traubenkernöl.

Diese Öle werden in den erfindungemäßen Haarbehandlungsmitteln in einer Gesamtmenge von 0,01 bis 3 Gew.-%, bevorzugt von 0,05 bis 2,5 Gew.-%, insbesondere von 0,1 bis 2 Gew.-%, bezogen auf das Gesamtgewicht des Haarbehandlungsmittels, eingesetzt.

Als konditionierende Komponenten können erfindungsgemäß ebenfalls kationische Polymere eingesetzt werden. Unter erfindungsgemäß geeigneten kationischen Polymeren sind Polymere zu verstehen, welche in der Haupt- und/oder Seitenkette "temporär kationische" oder "permanent kationische" Gruppen aufweisen. Als "permanent kationisch" werden erfindungsgemäß solche Polymere bezeichnet, welche unabhängig vom pH-Wert des Mittels eine kationische Gruppe aufweisen. Dies sind in der Regel Polymere, welche ein quartäres Stickstoffatom, beispielsweise in Form einer Ammoniumgruppe, enthalten.

Bevorzugte kationische Gruppen enthalten quartäre Ammoniumgruppen. Insbesondere solche Polymere, bei welchen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (III), 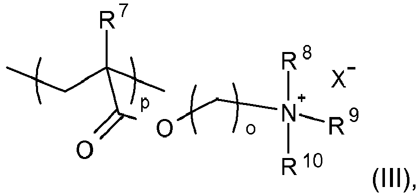 in welcher
- R⁷: -H oder-CH₃ ist,
- R⁸, R⁹ und R¹⁰: jeweils unabhängig voneinander, ausgewählt sind aus C₁₋₄-Alkyl-, -Alkenyl- oder -Hydroxyalkylgruppen,
- o: 1, 2, 3 oder 4,
- p: eine natürliche Zahl und
- X⁻: ein physiologisch verträgliches organisches oder anorganisches Anion ist, sowie Copolymere,
bestehend im Wesentlichen aus den in Formel (III) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für welche mindestens eine der folgenden Bedingungen gilt:
- R⁷ steht für eine Methylgruppe
- R⁸, R⁹ und R¹⁰ stehen für Methylgruppen
- o hat den Wert 2.

Als physiologisch verträgliches Gegenionen X- kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Erfindungsgemäß geeignete Homo- oder Copolymere, welche sich von der Formel (III) ableiten, sind beispielsweise unter den Handelsbezeichnungen Salcare® SC 95, Salcare® SC 96 und Salcare® SC 92 im Handel erhältlich.

Weitere bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR® 400 sind bevorzugte quaternierte Cellulose-Derivate,
- hydrophob modifizierte Cellulosederivate, beispielsweise die unter dem Handelsnamen SoftCat® vertriebenen kationischen Polymere,
- kationische Alkylpolyglycoside,
- kationiserter Honig, beispielsweise das Handelsprodukt Honeyquat® 50,
- kationische Guar-Derivate, wie insbesondere die unter den Handelsnamen Cosmedia® Guar und Jaguar® vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning® 929 Emulsion (enthaltend ein hydroxylamino-modifiziertes Silikon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammonium-chlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden,
- quaternierter Polyvinylalkohol,
   sowie die unter den Bezeichnungen
- Polyquaternium-2,
- Polyquaternium-17,
- Polyquaternium -8 und
- Polyquaternium-27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft® LM 200) bekannten Polymere. Ebenfalls erfindungsgemäß verwendbar sind die Copolymere des Vinylpyrrolidons, wie sie als Handelsprodukte Copolymer 845 (Hersteller: ISP), Gaffix® VC 713 (Hersteller: ISP), Gafquat® ASCP 1011, Gafquat® HS 110, Luviquat® 8155 und Luviquat® MS 370 erhältlich sind.

Weitere erfindungsgemäße kationische Polymere sind die sogenannten "temporär kationischen" Polymere. Diese Polymere enthalten üblicherweise eine Aminogruppe, welche bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen® CMF, Hydagen® HCMF, Kytamer® PC und Chitolam® NB/101 im Handel verfügbar sind. Chitosane sind deacetylierte Chitine, welche in unterschiedlichen Deacetylierungsgraden und unterschiedlichen Abbaugraden (Molekulargewichten) im Handel erhältlich sind.

In einer besonders bevorzugten Ausführungsform der Erfindung enthalten die erfindungsgemäßen Haarbehandlungsmittel zur Steigerung der Hautkonditionierung mindestens ein kationisches Polymer, welches ausgewählt ist aus der Gruppe der kationischen Cellulose-Derivate, kationischen Guar-Derivate und/oder Polyquaternium-7 (Merquat 550), Polyquaternium-6, Polyquaternium-10 und/oder Polyquaternium-67 (SoftCat®-Polymere).

Das oder die kationische(n) Polymer(e) ist (sind) in den erfindungsgemäßen Haarbehandlungsmitteln in einer Gesamtmenge von 0,1 bis 5 Gew.-%, bezogen auf das Gesamtgewicht des Haarbehandlungsmittels, enthalten. Gesamtmengen von 0,2 bis 3 Gew.-%, vorzugsweise von 0,5 bis 2 Gew.-%, bezogen auf das Gesamtgewicht des Haarbehandlungsmittels, sind besonders bevorzugt.

Unter erfindungsgemäß geeigneten Pflanzenextrakten sind Extrakte zu verstehen, welche aus allen Teilen einer Pflanze hergestellt werden können.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Erfindungsgemäß sind vor allem die Extrakte aus Grünem Tee, Weißem Tee, Eichenrinde, Brennnessel, Hamamelis, Hopfen, Kamille, Klettenwurzel, Schachtelhalm, Weißdorn, Lindenblüten, Litschi, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Ginseng, Ingwerwurzel, Echinacea purpurea, Olea europea, Foeniculum vulgaris und Apim graveolens bevorzugt.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 bis 80 Gew.-% Aktivsubstanz und als Lösungsmittel das bei ihrer Gewinnung eingesetzte Extraktionsmittel oder Extra ktionsmittelgemisch.

Zusätzlich kann es sich als vorteilhaft erweisen, den erfindungsgemäßen Haarbehandlungsmitteln Feuchthaltemittel bzw. Penetrationshilfsstoffe und/oder Quellmittel zuzusetzen. Diese Hilfsstoffe sorgen für eine bessere Penetration von Wirkstoffen in die keratinische Faser oder helfen die keratinische Faser aufzuquellen. Hierzu zählen beispielsweise Harnstoff und Harnstoffderivate, Guanidin und dessen Derivate, Arginin und dessen Derivate, Wasserglas, Imidazol und Dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole, wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol. Erfindungsgemäß besonders geeignet ist Glycerin.

Die Feuchthaltemittel werden in den erfindungsgemäßen Haarbehandlungsmitteln in einer Gesamtmenge von 0,01 bis 10 Gew.-%, bevorzugt von 0,05 bis 5 Gew.-%, insbesondere von 0,1 bis 3 Gew.-%, bezogen auf das Gesamtgewicht des Haarbehandlungsmittels, eingesetzt.

Die erfindungsgemäßen Haarbehandlungsmittel eignen sich als kosmetische Zusammensetzungen zur Reinigung und Pflege der Haare und können beispielsweise als Haarshampoos, Haarkonditionierer, konditionierende Shampoos, Haarspülungen, Haarkuren, Haarpackungen, Haar-Tonics, Haarfärbeshampoos oder deren Kombinationen vorliegen. Sie weisen hervorragende Schaumeigenschaften, eine hohe Reinigungsleistung sowie eine hohe Lagerstabilität auf. Zusätzlich weist die Kombination aus dem speziellen Carbonsäure-N-alkylpolyhydroxyalkylamid und mindestens einem weiteren speziellen Tensid keine nachteiligen Eigenschaften in Bezug auf die Haareigenschaften, wie Kämmbarkeit oder Griff, auf. Darüber hinaus führt der Einsatz der Kombination nicht zu einer ungünstigen Wechselwirkung mit weiteren Pflegestoffen, so dass die Kämmbarkeit und der Griff durch Zusatz weiterer Pflegestoffe zusätzlich gesteigert werden kann. Darüber hinaus kommt es durch Einarbeitung der Kombination nicht zu einer verschlechterten Lagerstabilität. Neben den vorgenannten zwingenden erfindungsgemäßen Komponenten und den fakultativen, aber bevorzugten weiteren Komponenten können die erfindungsgemäßen Haarbehandlungsmittel weitere Stoffe enthalten, welche die Haare konditionieren oder die Anwendungseigenschaften der Mittel verbessern.

Gemäß einer bevorzugten Ausführungsform der vorliegenden Erfindung können die Reinigungsmittel zur Stabilisierung einen anorganischen Verdicker enthalten. Als anorganische Verdickungsmittel können im Rahmen der vorliegenden Erfindung Schichtsilikate (polymere, kristalline Natriumdisilicate) eingesetzt werden. Besonders stabile Schäume mit hervorragenden Schaumeigenschaften werden erhalten, wenn als anorganischer Verdicker Magnesium Aluminium Silikate aus der Gruppe der Bentonite, insbesondere Smektite, wie Montmorillonit oder Hectorit, welche gegebenenfalls auch geeignet modifiziert sein können, eingesetzt werden. Darüber hinaus können auch synthetische Schichtsilikate, wie beispielsweise das von der Firma Süd Chemie unter der Handelsbezeichnung Optigel vertriebene Magnesiumschichtsilikat verwendet werden.

Erfindungsgemäß einsetzbar sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Carbonsäuren mit Triethanolamin, quaternierte Estersalze von Carbonsäuren mit Diethanolalkylaminen und quaternierten Estersalzen von Carbonsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxy-ethyl)dimethylammoniumchlorid, sowie Dehyquart® F-75, Dehyquart® C-4046, Dehyquart® L80 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.

Die kationischen Tenside werden bevorzugt in einer Gesamtmenge von 0,05 bis 10 Gew.-%, bezogen auf das Gesamtgewicht des Haarbehandlungsmittels, eingesetzt. Eine Gesamtmenge von 0,1 bis 5 Gew.-%, bezogen auf das Gesamtgewicht des Haarbehandlungsmittels, ist besonders bevorzugt.

In einer weiteren bevorzugten Ausführungsform kann die Wirkung der erfindungsgemäßen Reinigungsmittel durch Emulgatoren gesteigert werden. Solche Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 Kohlenstoffatomen, an Fettsäuren mit 12 bis 22 Kohlenstoffatomen und an Alkylphenole mit 8 bis 15 Kohlenstoffatomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen, zum Beispiel das im Handel erhältliche Produkt Montanov® 68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 Kohlenstoffatomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospholipide, die z. B. als Lecithine bzw. Phosphatidylcholine aus z. B. Eidotter oder Pflanzensamen (z. B. Sojabohnen) gewonnen werden, verstanden.
- Fettsäureester von Zuckern und Zuckeralkoholen wie Sorbit,
- Polyglycerine und Polyglycerinderivate wie beispielsweise Polyglycerinpoly-12-hydroxystearat (Handelsprodukt Dehymuls® PGPH),
- Lineare und verzweigte Fettsäuren mit 8 bis 30 C - Atomen und deren Na-, K-, Ammonium-, Ca-, Mg- und Zn - Salze,
- Monoester und/oder Gemische aus Monoestern und Diestern des Glycerins mit verzweigten, oder geradkettigen, gesättigten oder ungesättigten Carbonsäuren einer Kohlenstoffkettenlänge von 8 bis 24, bevorzugt von 10 bis 18 und insbesondere von 12 bis 16, die einen Ethoxylierungsgrad von 1 bis 20, bevorzugt von 2 bis 17, besonders bevorzugt von 4 bis 13 und insbesondere von 6 bis 10 aufweisen. Erfindungsgemäß bevorzugt sind die ethoxylierten Glyceryloleate und Glycerylcocoate und insbesondere bevorzugt ist PEG-7 Glyceryl Cocoate, wie es beispielsweise im Handel unter der Bezeichnung Tegosoft® GC oder Cetiol® HE erhältlich ist.
Die Emulgatoren werden bevorzugt in einer Gesamtmenge von 0,1 bis 25 Gew.-%, insbesondere von 0,5 bis 15 Gew.-%, bezogen auf das Gesamtgewicht des Haarbehandlungsmittels, eingesetzt.

Prinzipiell können nichtionogene Emulgatoren mit einem HLB-Wert von 8 bis 18 eingesetzt werden. Nichtionogene Emulgatoren mit einem HLB-Wert von 10 bis 15 können erfindungsgemäß bevorzugt sein.

In einer weiteren Ausführungsform der Erfindung können die erfindungsgemäßen Haarbehandlungsmittel zur weiteren Unterstützung ihrer haut- und haarpflegenden Wirkung zudem Proteinhydrolysate und/oder deren Derivate enthalten. Proteinhydrolysate sind Produktgemische, welche durch sauer, basisch oder enzymatisch katalysierten Abbau von Proteinen (Eiweißen) erhalten werden.

Erfindungsgemäß können Proteinhydrolysate pflanzlichen und tierischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex), Kerasol® (Croda) und ProSina® (Croda) vertrieben.

Erfindungsgemäß bevorzugt sind Proteinhydrolysate pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin® (Cognis), DiaMin® (Diamalt), Lexein® (Inolex) und Crotein® (Croda) erhältlich.

Wenngleich der Einsatz der Proteinhydrolysate als solche bevorzugt ist, können an deren Stelle gegebenenfalls auch anderweitig erhaltene Aminosäuregemische oder einzelne Aminosäuren wie beispielsweise Arginin, Lysin, Histidin oder Pyrroglutaminsäure eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon® (Cognis), Gluadin® (Cognis), Lexein® (Inolex), Crolastin® (Croda) oder Crotein® (Croda) vertrieben.

Erfindungsgemäß einsetzbar sind auch kationisierte Proteinhydrolysate, wobei das zugrundeliegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder von biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder durch eine Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25.000 Dalton, bevorzugt 250 bis 5.000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein. Ganz besonders bevorzugt sind die kationischen Proteinhydrolysate und -derivate auf tierischer Basis.

Die Proteinhydrolysate und deren Derivate werden bevorzugt in einer Gesamtmenge von 0,01 bis 10 Gew.-%, bezogen auf das Gesamtgewicht des Haarbehandlungsmittels, eingesetzt. Eine Gesamtmenge von 0,1 bis 5 Gew.-%, vorzugsweise von 0,1 bis 3 Gew.-%, bezogen auf das Gesamtgewicht des Haarbehandlungsmittels, ist ganz besonders bevorzugt.

Ebenfalls als vorteilhaft hat sich die Kombination des erfindungsgemäßen Haarbehandlungsmittels mit Vitaminen, Provitaminen und Vitaminvorstufen sowie deren Derivaten erwiesen.

Dabei sind erfindungsgemäß solche Vitamine, Pro-Vitamine und Vitaminvorstufen bevorzugt, welche üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die Vitamin A-Komponente wird bevorzugt in einer Gesamtmenge von 0,05 bis 1 Gew.-%, bezogen auf das Gesamtgewicht des Haarbehandlungsmittels, eingesetzt.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in bevorzugt in einer Gesamtmenge von 0,05 bis 1 Gew.-%, bezogen auf das Gesamtgewicht des Haarbehandlungsmittels, eingesetzt wird.
- Vitamin B₅ (Pantothensäure und Panthenol). Im Rahmen dieser Gruppe wird bevorzugt das Panthenol eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 A1 offenbarten kationischen Panthenolderivate. Die genannten Verbindungen des Vitamin B₅-Typs werden bevorzugt in einer Gesamtmenge von 0,05 bis 10 Gew.-%, bezogen auf das Gesamtgewicht des Haarbehandlungsmittels, eingesetzt. Eine Gesamtmenge von 0,1 bis 5 Gew.-%, bezogen auf das Gesamtgewicht des Haarbehandlungsmittels, ist besonders bevorzugt.
- Vitamin B₆ (Pyridoxin sowie Pyridoxamin und Pyridoxal).

Vitamin C (Ascorbinsäure). Die übliche Gesamtmenge von Vitamin C beträgt 0,1 bis 3 Gew.-%, bezogen auf das Gesamtgewicht des Haarbehandlungsmittels. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, werden erfindungsgemäß bevorzugt in einer Gesamtmenge von 0,05 bis 1 Gew.-%, bezogen auf das Gesamtgewicht des Haarbehandlungsmittels, eingesetzt.

Vitamin F. Unter dem Begriff "Vitamin F" werden üblicherweise essentielle Carbonsäuren, insbesondere Linolsäure, Linolensäure und Arachidonsäure, verstanden.

Vitamin H. Als Vitamin H wird die Verbindung (3aS,4S, 6aR)-2-Oxohexahydrothienol[3,4-d]-imidazol-4-valeriansäure bezeichnet, für die sich aber zwischenzeitlich der Trivialname Biotin durchgesetzt hat. Biotin wird bevorzugt in einer Gesamtmenge von 0,0001 bis 1,0 Gew.-%, vorzugsweise von 0,001 bis 0,01 Gew.-%, bezogen auf das Gesamtgewicht des Haarbehandlungsmittels, eingesetzt.

Erfindungsgemäß besonders bevorzugt ist der Einsatz von Vitaminen, Provitaminen und Vitaminvorstufen aus den Gruppen A, B, E und H. Panthenol und seine Derivate sowie Nicotinsäureamid und Biotin sind besonders bevorzugt.

Weiterhin kann in einer bevorzugten Ausführungsform der Erfindung zusätzlich ein UV-Filter eingesetzt werden. Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB (280 - 315 nm)- oder im UVC (< 280 nm)-Bereich liegt. UV-Filter mit einem Absorptionsmaximum im UVB-Bereich, insbesondere im Bereich von etwa 280 bis etwa 300 nm, sind besonders bevorzugt.

Die UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Gemäß einer weiteren Ausführungsform der Erfindung sind solche UV-Filter bevorzugt, welche eine kationische Gruppe, insbesondere eine quartäre Ammoniumgruppe, aufweisen.

Zwei bevorzugte UV-Filter mit kationischen Gruppen sind die als Handelsprodukte erhältlichen Verbindungen Zimtsäureamidopropyl-trimethylammoniumchlorid (Incroquat® UV-283) und Dodecyldimethylaminobenzamidopropyldimethylammoniumtosylat (Escalol® HP 610).

Der oder die UV-Filter werden üblicherweise in einer Gesamtmenge von 0,1 bis 5 Gew.-%, bezogen auf das Gesamtgewicht des Haarbehandlungsmittels, eingesetzt. Eine Gesamtmenge von 0,4 bis 2,5 Gew.-%, bezogen auf das Gesamtgewicht des Haarbehandlungsmittels, ist bevorzugt.

Hinsichtlich der Art der erfindungsgemäßen kosmetischen Haarbehandlungsmittel bestehen keine prinzipiellen Einschränkungen. Als Konfektionierung dieser Zubereitungen sind beispielsweise Cremes, Lotionen, Lösungen, Wässer, Emulsionen wie W/O-, O/W-, PIT-Emulsionen (Emulsionen nach der Lehre der Phaseninversion, PIT genannt), Mikroemulsionen und multiple Emulsionen, grobe, instabile, ein oder mehrphasige Schüttelmixturen, Gele, Sprays, Aerosole und Schaumaerosole geeignet.

Die erfindungsgemäßen Haarbehandlungsmittel zeichnen sich neben den hervorragend reinigenden, pflegenden und schäumenden Eigenschaften weiterhin durch ihre anwendungs- und herstellungsfreundliche Rheologie und Viskosität aus.

Dazu weisen sie eine Viskosität im Bereich von 5.000 mPas bis 15.000 mPas, bevorzugt von 6.000 mPas bis 12.000 mPas, insbesondere von 7.500 mPas bis 10.500 mPas auf (jeweils gemessen mit einem Haake Viskosimeter Viscotester VT550; 20 °C, Messeinrichtung Zylinder MK-2; Schwergeschwindigkeit 8/sek.).

Neben den erfindungsgemäß zwingenden Komponenten und den weiteren, oben genannten bevorzugten Komponenten können prinzipiell alle weiteren, dem Fachmann für solche kosmetischen Zusammensetzungen bekannten Komponenten eingesetzt werden.

Weitere Wirk-, Hilfs- und Zusatzstoffe sind beispielsweise:
- Verdickungsmittel wie Gelatine oder Pflanzengumme, beispielsweise Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, vollsynthetische Hydrokolloide wie z. B. Polyvinylalkohol,
- Strukturanten wie Maleinsäure und Milchsäure,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- Farbstoffe zum Anfärben des Mittels,
- weitere Substanzen zur Einstellung des pH-Wertes, wie beispielsweise α- und β-Hydroxycarbonsäuren,
- Wirkstoffe wie Allantoin und Bisabolol,
- Komplexbildner wie EDTA, NTA, β-Alanindiessigsäure und Phosphonsäuren,
- Ceramide. Unter Ceramiden werden N-Acylsphingosin (Fettsäureamide des Sphingosins) oder synthetische Analogen solcher Lipide (sogenannte Pseudo-Ceramide) verstanden,
- Trübungsmittel wie Latex, Styrol/PVP- und Styrol/Acrylamid-Copolymere,
- Perlglanzmittel wie Ethylenglykolmono- und -distearat sowie PEG-3-distearat,
- Pigmente,
- Treibmittel wie Propan-Butan-Gemische, N₂O, Dimethylether, CO₂ und Luft,
- Viskositätsregler wie Salze (NaCl).

Ein zweiter Gegenstand der vorliegenden Erfindung ist eine Verwendung eines erfindungsgemäßen Haarbehandlungsmittels zur Reinigung und/oder Pflege von Kopfhaut und Haaren. Bezüglich der Verwendung des erfindungsgemäßen Haarbehandlungsmittels gilt mutatis mutandis das zu den erfindungsgemäßen Haarbehandlungsmitteln Gesagte.

Schließlich ist ein dritter Gegenstand der vorliegenden Erfindung ein Verfahren zur Reinigung und/oder Pflege von Kopfhaut und Haaren, wobei ein erfindungsgemäßes Haarbehandlungsmittel auf das trockene oder gegebenenfalls zuvor gewaschene und/oder nasse Haar aufgetragen und nach einer gewissen Zeitdauer gegebenenfalls ausgespült wird, wobei nach der Anwendung des Haarbehandlungsmittels eine verbesserte Trocken- und Nasskämmbarkeit sowie ein verbesserter Griff auftreten. Bezüglich des Verfahrens zur Reinigung und/oder Pflege von Kopfhaut und Haaren gilt mutatis mutandis das zu den erfindungsgemäßen Haarbehandlungsmitteln Gesagte.

Die folgenden Beispiele erläutern die Erfindung, ohne sie jedoch darauf zu beschränken.

### Beispiele:

Es wurden die folgenden Haarbehandlungsmittel hergestellt, wobei alle Zahlenwerte in der nachfolgenden Tabelle - sofern nicht anders angegeben - der Menge des jeweiligen Rohstoffs in Gewichtsprozent entsprechen. Das in den nachfolgenden Beispielen eingesetzte Carbonsäure-N-alkylpolyhydroxyalkylamid der Formel (I) ist bevorzugt eine Verbindung der Formel (la) oder (Ib) sowie deren Mischung.

**Tabelle 1: erfindungsgemäße Rezeptur**

| Rohstoff | Rezepturen | | | | | | | | |
|---|---|---|---|---|---|---|---|---|---|
| | I | II | III | IV | V | VI | VII | VIII | IX |
| Texapon N70 NA | 12 | | | | 12 | 12 | 12 | 12 | |
| Texapon OCN | | 6,0 | 10 | 10 | 4,0 | 4,0 | | | 10 |
| Dehyton K | 8,0 | 8,0 | | 8,0 | 6,0 | 6,0 | 8,0 | 8,0 | 8,0 |
| Plantacare® 818UP | 8,0 | 8,0 | 8,0 | | | | | | |
| Carbonsäure-N-alkylpolyhydroxyalkylamid der Formel (I) | 3,0 | 2,5 | 4,0 | 1,5 | 2,0 | 3,5 | 4,5 | 1,0 | 5,0 |
| Cutina® HR | 0,4 | 0,4 | 0,4 | 0,4 | 0,4 | 0,4 | 0,4 | 0,4 | 0,4 |
| Cetiol® HE | 1,0 | 1,0 | 1,0 | 1,0 | 1,0 | 1,0 | 1,0 | 1,0 | 1,0 |
| Polymer JR 400 | 0,3 | 0,3 | 0,3 | 0,3 | 0,3 | 0,3 | 0,3 | 0,3 | 0,3 |
| Arlypon F | 1,2 | 1,2 | 1,2 | 1,2 | 1,2 | 1,2 | 1,2 | 1,2 | 1,2 |
| ProSina® | 0,3 | 0,3 | 0,3 | 0,3 | 0,3 | 0,3 | 0,3 | 0,3 | 0,3 |
| Citronensäure | 0,5 | 0,5 | 0,5 | 0,5 | 0,5 | 0,5 | 0,5 | 0,5 | 0,5 |
| Natriumchlorid | 1,3 | 1,3 | 1,3 | 1,3 | 1,3 | 1,3 | 1,3 | 1,3 | 1,3 |
| Parfüm | q.s | q.s | q.s | q.s | q.s | q.s | q.s | q.s | q.s |
| Konservierung | q.s. | q.s. | q.s. | q.s. | q.s. | q.s. | q.s. | q.s. | q.s. |
| Wasser | ad 100 | ad 100 | ad 100 | ad 100 | ad 100 | ad 100 | ad 100 | ad 100 | ad 100 |

Es wurden die folgenden Handelsprodukte eingesetzt:
- Texapon® N70 NA:: INCI-Bezeichnung: Sodium Laureth Sulfate; BASF
- Texapon® OCN:: INCI-Bezeichnung: Sodium Lauryl Sulfate; BASF
- Dehyton® K:: INCI-Bezeichnung: Cocoamidopropyl betain: BASF
- Plantacare® 818UP:: INCI-Bezeichnung: Coco-Glucoside, ca. 50 % Aktivsubstanz; Cognis
- Cutina® HR:: INCI-Bezeichnung: Hydrogenated Castor Oil, BASF
- Cetiol® HE:: INCI-Bezeichnung: PEG-7 Glyceryl Cocoate; BASF
- Polymer JR 400®:: INCI-Bezeichnung: Polyquaternium-10, UNION CARBIDE
- Arlypon® F:: INCI-Bezeichnung: Laureth-2; BASF
- ProSina®:: INCI-Bezeichnung: Hydrolyzed keratin; Croda

Die Herstellung der Rezepturen I bis IX erfolgt nach folgender allgemeiner Vorschrift:
Die Waschrohstoffe sowie die Hauptmenge des Wassers werden vorgelegt, auf 70 °C bis 80 °C erwärmt und bis zur Homogenität verrührt. Anschließend werden gegebenenfalls die in warmem Wasser vorgequollenen Verdicker sowie polymeren Pflegestoffe hinzugefügt. Nachdem alle Inhaltsstoffe des Haarbehandlungsmittels hinzugefügt sind, kann gegebenenfalls eine Begasung mit Gas unter Kühlung und Rühren auf 25 °C erfolgen. Ist keine Begasung vorgesehen, wird die Mischung unter Rühren auf 25 °C abgekühlt. Die Konservierungsmittel sowie gegebenenfalls mit einem Lösungsvermittler vermischte Parfümöle werden erst bei einer Temperatur von 35 °C zugegebenen.

### Ergebnisse:

Die erfindungsgemäßen Rezepturen I bis IX resultieren in Haarbehandlungsmitteln, welche ein hohes Schaumvermögen und eine hervorragende Reinigungsleistung aufweisen. Der Einsatz der Tensidkombination aus speziellem eingesetzte Carbonsäure-N-alkylpolyhydroxyalkylamid und mindestens einem weiteren Tensid führt darüber hinaus zu einer Verbesserung der Haareigenschaften, wie Kämmbarkeit und Griff, und weist keine negativen Wechselwirkungen mit weiteren Pflege- und Inhaltstoffen auf.

Die mit den erfindungsgemäßen Haarbehandlungsmitteln gereinigten Haare weisen eine verbesserte Trocken- und Nasskämmbarkeit sowie einen verbesserten Griff auf.

Weiterhin weisen die erfindungsgemäßen Haarbehandlungsmittel eine hohe Lagerstabilität auf.

## Patentansprüche

1. Haarbehandlungsmittel, enthaltend:
a) mindestens ein Carbonsäure-N-alkylpolyhydroxyalkylamid der Formel (I) 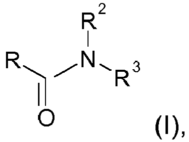 worin
R für einen aliphatischen Alkylrest mit 16 bis 18 Kohlenstoffatomen steht,
R² für einen Alkylrest oder einen Hydroxyalkylrest mit 1 bis 4 Kohlenstoffatomen steht,
R³ für einen linearen oder verzweigten Polyhydroxyalkylrest mit 3 bis 12 Kohlenstoffatomen und 3 bis 10 Hydroxylgruppen steht, und
b) mindestens ein weiteres Tensid, ausgewählt aus der Gruppe der
(i) Alkylethersulfate mit 8 bis 18 Kohlenstoffatomen und 1 bis 10 Oxyethylgruppen;
(ii) Alkylsulfate mit 8 bis 18 Kohlenstoffatomen;
(iii) Alkylpolyglucoside;
(iv) amphoteren Tenside;
sowie deren Mischungen.

2. Haarbehandlungsmittel nach Anspruch 1, wobei das Haarbehandlungsmittel mindestens ein Carbonsäure-N-alkylpolyhydroxyalkylamid der Formel (la) und/oder der Formel (Ib) 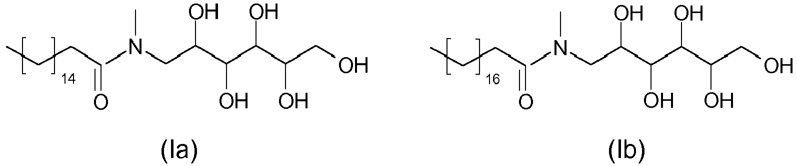 enthält.

3. Haarbehandlungsmittel nach einem der Ansprüche 1 oder 2, wobei das Haarbehandlungsmittel das mindestens eine Carbonsäure-N-alkylpolyhydroxyalkylamid der Formel (I) in einer Gesamtmenge von 0,01 bis 30 Gew.-%, vorzugsweise von 0,1 bis 20 Gew.-%, bevorzugt von 0,3 bis 15 Gew.-%, weiter bevorzugt von 0,5 bis 10 Gew.-%, noch weiter bevorzugt von 0,7 bis 8 Gew.-%, insbesondere von 1,0 bis 5,0 Gew.-%, bezogen auf das Gesamtgewicht des Haarbehandlungsmittels, enthält.

4. Haarbehandlungsmittel nach einem der vorangehenden Ansprüche, wobei das mindestens eine weitere Tensid ausgewählt ist der Gruppe der
(i) Natriumlaurethsulfate;
(ii) Natriumlaurylsulfate;
(iii) Alkylpolyglucoside der Formel
R⁴O-[G]ₚ
in welcher
R⁴ für einen Alkyl- und/oder Alkenylrest mit 4 bis 22 Kohlenstoffatomen,
G für einen Zuckerrest mit 5 oder 6 Kohlenstoffatomen und
p für Zahlen von 1 bis 10 steht;
(iv) C₈₋₁₈-Alkylamido(C₁₋₄)-alkylbetaine;
sowie deren Mischungen.

5. Haarbehandlungsmittel nach einem der vorangehenden Ansprüche, wobei das mindestens eine weitere Tensid in einer Gesamtmenge von 0,01 bis 50 Gew.-%, vorzugsweise von 0,1 bis 40 Gew.-%, bevorzugt von 2 bis 30 Gew.-%, weiter bevorzugt von 5 bis 20 Gew.-%, insbesondere von 8 bis 15 Gew.-%, bezogen auf das Gesamtgewicht des Haarbehandlungsmittels, enthalten ist.

6. Haarbehandlungsmittel nach einem der vorangehenden Ansprüche, wobei das Haarbehandlungsmittel ein Gewichtsverhältnis der Gesamtmenge des mindestens einen Carbonsäure-N-alkylpolyhydroxyalkylamids (a) zu der Gesamtmenge des mindestens einen weiteren Tensids (b) von 1 : 15 bis 1 : 1, vorzugsweise von 1 : 12 bis 1 : 2, bevorzugt von 1 : 8 bis 1 : 2,5, insbesondere von 1 : 6 bis 1 : 3, enthält.

7. Haarbehandlungsmittel nach einem der vorangehenden Ansprüche, wobei das Haarbehandlungsmittel weiterhin mindestens einen haar- und/oder hautkonditionierenden Wirkstoff enthält, wobei der Wirkstoff ausgewählt ist aus der Gruppe der Ölkomponenten, kationischen Polymere, Pflanzenextrakte und/oder Feuchthaltemittel.

8. Verwendung eines Haarbehandlungsmittels nach einem der Ansprüche 1 bis 7 zur Reinigung und/oder Pflege von Kopfhaut und Haaren.

9. Verfahren zur Reinigung und/oder Pflege von Kopfhaut und Haaren, wobei ein Haarbehandlungsmittel nach einem der Ansprüche 1 bis 7 auf das trockene oder gegebenenfalls zuvor gewaschene und/oder nasse Haar aufgetragen und nach einer gewissen Zeitdauer gegebenenfalls ausgespült wird, wobei nach der Anwendung des Haarbehandlungsmittels eine verbesserte Trocken- und Nasskämmbarkeit sowie ein verbesserter Griff auftreten.
